# EUROPEAN PATENT SPECIFICATION

(11) **EP 3 216 450 B1**
(45) Date of publication and mention of the grant of the patent: **22.09.2021**
(21) Application number: 15857367.5
(22) Date of filing: 06.11.2015
(51) Int. Cl.: A61K 9/20, A61K 9/28, A61K 9/48, A61K 31/519, A61P 35/00, A61P 43/00

(54) **PHARMACEUTICAL PREPARATION COMPRISING CYCLIN INHIBITOR AND PREPARATION METHOD THEREOF**
PHARMAZEUTISCHE ZUBEREITUNG MIT CYCLINHEMMER UND HERSTELLUNGSVERFAHREN DAFÜR
PRÉPARATION PHARMACEUTIQUE COMPRENANT UN INHIBITEUR DE LA CYCLINE ET SON PROCÉDÉ DE PRÉPARATION

(30) Priority: 07.11.2014 CN 201410623810
(43) Date of publication of application: 13.09.2017
(73) Proprietor: Jiangsu Hansoh Pharmaceutical Group Co., Ltd., Jiangsu 222047 (CN)
(72) Inventor: ZENG, Jin, Lianyungang Jiangsu 222047 (CN); WANG, Ruijun, Lianyungang Jiangsu 222047 (CN); WANG, Xiaolei, Lianyungang Jiangsu 222047 (CN)
(74) Representative: Pistolesi, Roberto
(86) International application number: PCT/CN2015/093953
(87) International publication number: WO 2016/070834

(56) References cited:
- WO-A1-2014/128588
- WO-A1-2016/030439
- WO-A1-2016/193860
- CN-A- 101 001 857
- CN-A- 104 892 604
- CN-A- 105 213 322
- US-A1- 2005 059 670
- US-B2- 6 936 612
- Anonymous: "HIGHLIGHTS OF PRESCRIBING INFORMATION. IBRANCE (palbociclib) capsules, for oral use.", , February 2015 (2015-02), pages 1-20, XP055662235, Retrieved from the Internet: URL:https://www.accessdata.fda.gov/drugsat fda_docs/label/2015/207103s000lbl.pdf [retrieved on 2020-01-27]

## Description

### FIELD OF THE INVENTION

The present invention belongs to the field of pharmaceutical preparation, and specifically relates to a pharmaceutical preparation for use as a cyclin inhibitor and a preparation method thereof.

### BACKGROUND OF THE INVENTION

Cyclin-dependent kinase (CDK) has 13 members in total, which all belong to the serine/threonine protein kinase family, and has key functions such as promoting the phase transition of cell cycle, initiating DNA synthesis and regulating cell transcription and the like, depending on the combination with cyclin.

CDKs play a key role in the proliferation and death of all cells including healthy cells and tumor cells. Broad-spectrum CDK inhibitors can hardly exhibit high therapeutic window on patients, especially on patients who have not undergone a gene screening. The toxicity will be severe when the dosage is too high, while the efficacy will be negligible when the dosage is too low. Therefore, it is very important to selectively inhibit some CDKs. Of course, since most of the CDK subtypes have relatively similar chemical structures, how to improve the selectivity of CDK inhibitors is another challenge.

The advantages of using CDK4/6 as an anti-tumor target are as follows: (1) the inhibitors of CDK4/6 do not exhibit cytotoxicities of "pan-CDK inhibitors", such as myelosuppression and intestinal responses; (2) the increase of cell cyclin D level or the inactivation of P161NK4a can improve the sensitivity of cells to drugs, the aforementioned phenomena are presented in tumor cells relative to normal cells, therefore the targeted property of drugs will be increased to a certain extent.

The compound of formula I is a targeted CDK4/6 inhibitor, which can selectively inhibit cyclin-dependent kinase 4 and 6 (CDK4/6), restore cell cycle control, and block the proliferation of tumor cells. It acts on MDA-MB-435 breast cancer cells, and can effectively reduce the phosphorylation of RB on Ser780 and Ser795 site, and the IC₅₀ are 66 nM and 63 nM respectively.

Breast cancer is one of the most common malignant tumors of women, with high incidence rate and invasiveness, but the course of progress is slow. The data published by the International Cancer Research Center showed that about 1.67 million cases were newly diagnosed around the world in 2012, which accounted for 25% of all cancers. According to the Survey of Epidemiology and End Results (SEER) of US National Cancer Institute, the estimated incidence rate of breast cancer is 123.8/100 thousand people in U.S. in 2013, and the incidence rate of breast cancer of Asian female is 94.5/100 thousand people in 2005-2009. There is no official data in China, it is estimated that the estrogen or progesterone receptor is positive among 60%-70% of breast cancer patients in China, the incidence rate of estrogen receptor positive breast cancer is thus calculated to be 56.7-66.15/100 thousand people in China in 2005-2009. According to Thomson's prediction, the sales of this product will increase significantly after it comes into the market, and the sales will reach 2.027 billion US dollars in 2019. Pharmaceutical compositions, comprising the compound of formula I or the salt thereof, and a pharmaceutically acceptable excipient are disclosed in US 6 936 612 B2, US 2005/059670 A1, WO 2014/128588 A1. Palbociclib capsules for oral use, sold under the brand name Ibrance® among others, have an initial U.S. approval date in 2015.

### SUMMARY OF THE INVENTION

The objective of present invention is to provide a pharmaceutical preparation comprising 6-acetyl-8-cyclopentyl-5-methyl-2-(5-piperazin-1-yl-pyridin-2-yl-amino)-8H-pyrido[2,3-d]pyrimidin-7-one and a salt thereof, and a preparation method thereof.

The technical solution of the present invention is achieved as follows:
A pharmaceutical composition, comprising the compound of formula I or the salt thereof, and a pharmaceutically acceptable excipient as a carrier, wherein the salt comprises hydrochloride, isethionate, characterized in that in the pharmaceutical composition, the weight percentage of each component is as follows:

| | |
|---|---|
| compound I or salt thereof | 20~40% |
| disintegrant | 3~15% |
| diluent | 55~70% |
| lubricant | 0.1~3.5% |
| binder | 0~8% |
| surfactant | 0~5.0%. |

wherein the diluent is lactose and microcrystalline cellulose, and the weight ratio of lactose to microcrystalline cellulose is 1:2-2:1, preferably 1:1. the binder is at least one selected from the group consisting of hydroxypropyl cellulose and povidone, the disintegrant is at least one selected from the group consisting of crospovidone, sodium carboxymethyl starch, and croscarmellose sodium. the lubricant is at least one selected from the group consisting of magnesium stearate, colloidal silica, talc, and silica, the surfactant is sodium dodecyl sulfate.

On another aspect, the present invention provides a preparation method of the compound of formula I or the salt thereof. The preparation process comprises wet granulation, dry granulation, direct mixing and the like, and the dosage form comprises tablet, capsule.

Preferably, the composition is obtained by wet granulation, and the method comprises the following steps of:
(1) pre-mixing the compound of formula I or the salt thereof with a part of lubricant in a wet mixing granulator to obtain a pre-mixture;
(2) adding a granulation liquid to granulate the pre-mixture obtained in step (1), preferably, the granulation liquid being water;
(3) drying the granule obtained in step (2) in a fluidized bed dryer or a drying oven;
(4) optionally, dry screening the dry granule obtained in step (3);
(5) mixing the dry granule obtained in step (4) with the rest of excipient to obtain a final mixture;
(6) optionally, filling the mixture obtained in the aforementioned step (5) by a suitable capsule filling machine to prepare acapsule;
(7) optionally, pressing the mixture obtained in the aforementioned step (5) by a suitable tabletting machine to obtain a tablet core;
(8) optionally, film coating the tablet core obtained in step (7) with a film coating.

Preferably, the composition is obtained by dry granulation, and the method comprises the following steps of:
(1) mixing the compound of formula I or the salt thereof with the majority of excipient including a binder in a hopper mixer to obtain a pre-mixture;
(2) pressing the mixture obtained in step (1) in a suitable roller press machine;
(3) crushing the ribbon obtained during step (2) into a granule by a suitable grinding or screening step;
(4) optionally, mixing the granule obtained in step (3) with the rest of excipient in a mixer to obtain a final mixture;
(5) optionally, filling the mixture obtained in the aforementioned step (4) by a suitable capsule filling machine to prepare a capsule;
(6) optionally, pressing the mixture obtained in the aforementioned step (4) by a suitable tabletting machine to obtain a tablet core;
(7) optionally, film coating the tablet core obtained in step (6) with film a coating.

The present invention provides a pharmaceutical preparation of 6-acetyl-8-cyclopentyl-5-methyl-2-(5-piperazin-1-yl-pyridin-2-yl-amino)-8H-pyrido[2,3-d]pyrimid in-7-one and a salt thereof, which is stable and suitable for medical application. The pharmaceutical preparation has excellent dissolution behavior and good stability, which meets the requirement of clinical application, and enables the active ingredient to achieve a good *in vivo* bioavailability.

### DETAILED DESCRIPTION OF THE INVENTION

The embodiments of the present invention will be described in detail with reference to specific examples. The following examples are only intended to illuminate the present invention, and should not be considered as limiting the scope of the present invention. The compositions designated as Formula 2, 5, 6, 10, 14, 15, 16, 17, 18, 21, 30, 39, 40 and 41 are reference compositions. Compositions, wherein the diluent does not consist of microcrystalline cellulose and lactose, e.g. Formula 2 and Formula 14, or wherein an excipient is present in an amount which is not comprised by the range specified in claim 1, e.g. Formula 5 and Formula 6, do not form part of the invention.

### Example 1

### 1.1 Composition of unit formula

| | Formula 1 | Formula 2 | Formula 3 | Formula 4 | Formula 5 | Formula 6 | Formula 7 | Formula 8 |
|---|---|---|---|---|---|---|---|---|
| Compound of formula I | 75 | 75 | 75 | 75 | 75 | 75 | 100 | 125 |
| Microcrystalline cellulose | 35.2 | / | 28.2 | 54.3 | 18.8 | 37.6 | 93.9 | 117.3 |
| Lactose | 35.2 | 56.4 | 56.4 | 28.2 | 10.8 | 18.8 | 46.9 | 58.7 |
| Starch | 35.2 | 28.2 | / | / | / | / | / | / |
| Hydroxypropyl cellulose | 10.5 | 11.5 | 10.5 | / | / | 7.0 | 14 | 17.5 |
| Povidone | / | 20.0 | / | 10.5 | 3.5 | / | / | / |
| Sodium carboxymethyl starch | 10.5 | 5.5 | 31.5 | / | / | 21 | 14 | 17.5 |
| Crospovidone | / | 5.0 | / | 31.5 | / | / | / | / |
| Croscarmellose sodium | / | / | / | / | 10.5 | / | / | / |
| Sodium dodecyl sulfate | 4.2 | 4.2 | 4.2 | 6.3 | 0 | 2.8 | 5.6 | 7.0 |
| Talc | 2.1 | 2.1 | 2.1 | 2.1 | 0.7 | 1.4 | 2.8 | 3.5 |
| Magnesium stearate | 2.1 | 2.1 | 2.1 | 2.1 | 0.7 | 1.4 | 2.8 | 3.5 |
| Weight | 210 | 210 | 210 | 210 | 120 | 165 | 280 | 350 |

| | | | | | | | | |
|---|---|---|---|---|---|---|---|---|
| Note: "/" represents that the corresponding component is not added. | | | | | | | | |

### 1.2 Preparation

The compound of formula I and other excipients except magnesium stearate with the aforementioned formulation amount for 1000 tablets were mixed in a hopper mixer; a wetting agent was added to carry out wet granulation. The granule was dried in a fluidized bed at 45°C for 10 min, and sieved through a 1.0 mm sieve. Magnesium stearate was added, and the mixture was blended for 10 min. The content was monitored on line. The mixture was filled in capsules, or pressed into tablets.

### 1.3 Dissolution data

The dissolution rate of each formula in 0.1 mol/L HCl is shown in the table below.

| | Formula 1 | Formula 2 | Formula 3 | Formula 4 | Formula 5 | Formula 6 | Formula 7 | Formula 8 |
|---|---|---|---|---|---|---|---|---|
| 10 min | 35 | 40 | 32 | 27 | 22 | 42 | 41 | 37 |
| 15 min | 62 | 66 | 68 | 56 | 55 | 66 | 69 | 65 |
| 30 min | 78 | 82 | 77 | 70 | 73 | 79 | 82 | 79 |
| 45 min | 89 | 91 | 93 | 83 | 80 | 87 | 89 | 83 |
| 60 min | 95 | 96 | 98 | 88 | 91 | 99 | 96 | 95 |

### Example 2

### 2.1 Composition of unit formula

| | Formula 9 | Formula 10 | Formula 11 | Formula 12 |
|---|---|---|---|---|
| Compound of formula I | 75 | 75 | 100 | 125 |
| Microcrystalline cellulose | 38.7 | 10.8 | 93.9 | 117.3 |
| Lactose | 54.3 | 18.8 | 49.7 | 65.7 |
| Hydroxypropyl cellulose | / | / | / | 17.5 |
| Povidone | 10.5 | 3.5 | 14 | / |
| Sodium carboxymethyl starch | / | 10.5 | 14 | 17.5 |
| Croscarmellose sodium | 21 | / | / | / |
| Sodium dodecyl sulfate | 2.1 | / | 2.8 | / |
| Talc | 2.1 | 0.7 | 2.8 | 3.5 |
| Magnesium stearate | 2.1 | 0.7 | 2.8 | 3.5 |
| Weight | 210 | 120 | 280 | 350 |

| | | | | |
|---|---|---|---|---|
| Note: "/" represents that the corresponding component is not added. | | | | |

### 2.2 Preparation

The compound of formula I and other excipients except magnesium stearate with the aforementioned formulation amount for 1000 tablets were mixed in a hopper mixer. The mixture was pressed into a ribbon by using a roller press machine, the ribbon was then crushed into a granule. Magnesium stearate was added, and the mixture was blended for 10 min. The content was monitored on line. The mixture was filled in capsules, or pressed into tablets.

### 2.3 Dissolution data

| | Formula 9 | Formula 10 | Formula 11 | Formula 12 |
|---|---|---|---|---|
| 10 min | 44 | 38 | 35 | 38 |
| 15 min | 69 | 63 | 64 | 70 |
| 30 min | 76 | 74 | 75 | 81 |
| 45 min | 93 | 90 | 89 | 90 |
| 60 min | 98 | 98 | 93 | 92 |

### Example 3

### 3.1 Composition of unit formula

| | Formula 13 | Formula 14 | Formula 15 |
|---|---|---|---|
| Compound of formula I | 75 | 75 | 75 |
| Microcrystalline cellulose | 71.8 | / | 50 |
| Lactose | 35.9 | 25.8 | 15.7 |
| Hydroxypropyl cellulose | 10.5 | 6.0 | 9.0 |
| Sodium carboxymethyl starch | 10.5 | 6.0 | 5.0 |
| Crospovidone | / | 3.0 | 4.0 |
| Sodium dodecyl sulfate | 2.1 | / | 2.1 |
| Talc | 2.1 | 2.1 | 2.1 |
| Magnesium stearate | 2.1 | 2.1 | 2.1 |
| Weight | 210 | 120 | 165 |

| | | | |
|---|---|---|---|
| Note: "/" represents that the corresponding component is not added. | | | |

### 3.2 Preparation

The compound of formula I and excipients with the aforementioned formulation amount for 1000 tablets were mixed in a hopper mixer. The content was monitored on line. The mixture was filled in capsules, or pressed into tablets.

### 3.3 Dissolution data

| | Formula 13 | Formula 14 | Formula 15 |
|---|---|---|---|
| 10 min | 40 | 29 | 35 |
| 15 min | 71 | 56 | 64 |
| 30 min | 80 | 70 | 77 |
| 45 min | 93 | 78 | 85 |
| 60 min | 98 | 82 | 90 |

### Example 4

### 4.1 Composition of unit formula

| | Formula 16 | Formula 17 | Formula 18 |
|---|---|---|---|
| Active pharmaceutical ingredient (API)² | 125 | 125 | 125 |
| Microcrystalline cellulose | 124.3 | 64.5 | 27.1 |
| Lactose | 62.2 | 64.6 | 27.1 |
| Hydroxypropyl cellulose | 17.5 | 8.4 | 16.8 |
| Crospovidone | / | 14 | 10.5 |
| Croscarmellose sodium | 17.5 | / | / |
| Magnesium stearate | 3.5 | 3.5 | 3.5 |
| Weight | 350 | 280 | 210 |

| | | | |
|---|---|---|---|
| Note 1: "/" represents that the corresponding component is not added. Note 2: API represents hydrochloride or isethionate of the compound of formula I. | | | |

### 4.2 Preparation

The API and other excipients except magnesium stearate with the aforementioned formulation amount for 1000 tablets were mixed in a hopper mixer; a wetting agent was added to carry out wet granulation. The granule was dried in a fluidized bed at 45°C for 10 min, and sieved through a 1.0 mm sieve. Magnesium stearate was added, and the mixture was blended for 10 min. The content was monitored on line. The mixture was filled in capsules, or pressed into tablets.

### 4.3 Dissolution data

| | Formula 16 | Formula 17 | Formula 18 |
|---|---|---|---|
| 5 min | 70 | 73 | 71 |
| 15 min | 95 | 92 | 91 |
| 30 min | 98 | 97 | 98 |

### Example 5

### 5.1 Composition of unit formula

| | Formula 19 | Formula 20 | Formula 21 | Formula 22 |
|---|---|---|---|---|
| API² | 75 | 75 | 75 | 100 |
| Microcrystalline cellulose | 74.6 | 37.3 | 34.9 | 74.6 |
| Lactose | 37.3 | 74.6 | 34.9 | 74.6 |
| Povidone | 10.5 | 10.5 | 8.25 | 14 |
| Sodium carboxymethyl starch | / | 10.5 | 5 | 14 |
| Croscarmellose sodium | 10.5 | / | 3.25 | / |
| Magnesium stearate | 2.1 | 2.1 | 1.65 | 2.8 |
| Weight | 210 | 210 | 165 | 280 |

| | | | | |
|---|---|---|---|---|
| Note 1: "/" represents that the corresponding component is not added. Note 2: API represents hydrochloride of the compound of formula I (Note: the aforementioned formula is also applicable to isethionate, and the inventor detected that the dissolution effect of isethionate is very close to that of hydrochloride used as the API). | | | | |

### 5.2 Preparation

The API and other excipients except magnesium stearate with the aforementioned formulation amount for 1000 tablets were mixed in a hopper mixer. The mixture was pressed into a ribbon by using a roller press machine, the ribbon was then crushed into granules. Magnesium stearate was added, and the mixture was blended for 10 min. The content was monitored on line. The mixture was filled in capsules, or pressed into tablets.

### 5.3 Dissolution data

| | Formula 19 | Formula 20 | Formula 21 | Formula 22 |
|---|---|---|---|---|
| 5 min | 76 | 70 | 68 | 69 |
| 15 min | 99 | 94 | 97 | 91 |
| 30 min | 98 | 99 | 99 | 97 |

### Example 6

### 6.1 Composition of unit formula

| | Formula 23 | Formula 24 | Formula 25 |
|---|---|---|---|
| API² | 75 | 100 | 125 |
| Microcrystalline cellulose | 37.3 | 49.7 | 62.2 |
| Lactose | 74.6 | 99.5 | 124.3 |
| Hydroxypropyl cellulose | 10.5 | 14 | 17.5 |
| Croscarmellose sodium | 10.5 | 14 | 17.5 |
| Magnesium stearate | 2.1 | 2.8 | 3.5 |
| Weight | 210 | 280 | 350 |

| | | | |
|---|---|---|---|
| Note 1: "/" represents that the corresponding component is not added. Note 2: API represents hydrochloride of the compound of formula I (Note: the aforementioned formula is also applicable to isethionate, and the inventor detected that the dissolution effect of isethionate is very close to that of hydrochloride used as the API). | | | |

### 6.2 Preparation

The API and excipients with the aforementioned formulation amount for 1000 tablets were mixed in a hopper mixer. The content was monitored on line. The mixture was filled in capsules, or pressed into raw tablets, which were then coated. The weight increase of coating was controlled by 3%.

### 6.3 Dissolution data

| | Formula 23 | Formula 24 | Formula 25 |
|---|---|---|---|
| 5 min | 62 | 60 | 59 |
| 15 min | 90 | 90 | 94 |
| 30 min | 97 | 95 | 99 |

### Example 7

### 7.1 Unit dosage formula

| | Formula 26 | Formula 27 | Formula 28 | Formula 29 | Formula 30 |
|---|---|---|---|---|---|
| Salt of the compound of formula I¹ | 81 | 81 | 81 | 96 | 160 |
| Microcrystalline cellulose | 47.7 | 72.9 | 106.5 | 99 | 67 |
| Lactose | 47.7 | 72.9 | 106.5 | 99 | 67 |
| Hydroxypropyl cellulose | 10.5 | 13.5 | 17.5 | 17.5 | 17.5 |
| Croscarmellose sodium | 10.5 | 13.5 | 17.5 | 17.5 | 17.5 |
| Sodium dodecyl sulfate | 6.3 | 8.1 | 10.5 | 10.5 | 10.5 |
| Colloidal silica | 4.2 | 5.4 | 7 | 7 | 7 |
| Magnesium stearate | 2.1 | 2.7 | 3.5 | 3.5 | 3.5 |
| Weight | 210 | 270 | 350 | 350 | 350 |

| | | | | | |
|---|---|---|---|---|---|
| Note 1: the conversion coefficient of the compound of formula I to hydrochloride is 447.53/484.03=92.4%, i.e., 75 mg of the compound of formula I corresponds to 81 mg of hydrochloride of the compound of formula I; the conversion coefficient of the compound of formula I to isethionate is 447.53/573.53=78.0%, i.e., 75 mg of compound of formula I corresponds to 96 mg of isethionate of the compound of formula I; other specifications are converted correspondingly. | | | | | |

### 7.2 Preparation

For formulas 26-28, the API and excipients with the formulation amount for 1000 tablets were mixed in a hopper mixer. The content was monitored on line. The mixture was filled in capsules. For formulas 29-30, after mixing, the mixture was directly pressed into raw tablets, which were then coated. The weight increase of coating was controlled by 3%.

### 7.3 Dissolution data

| | Formula 26 | Formula 27 | Formula 28 | Formula 29 | Formula 30 |
|---|---|---|---|---|---|
| 5 min | 40 | 60 | 82 | 85 | 40 |
| 15 min | 82 | 85 | 95 | 95 | 73 |
| 30 min | 97 | 100 | 103 | 98 | 99 |

### Example 8

### 8.1 Unit dosage formula

| | | | | | | | Formula 31 | Formula 32 | Formula 33 | Formula 34 | Formula 35 |
|---|---|---|---|---|---|---|---|---|---|---|---|
| Compound of formula I | | | | | | | 75 | 100 | 100 | 125 | 125 |
| Microcrystalline cellulose | | | | | | | 86.7 | 77.1 | 154.1 | 144.5 | 56.3 |
| Lactose | | | | | | | 86.7 | 154.1 | 77.1 | 144.5 | 232.7 |
| Sodium carboxymethyl starch | | | | | | | 13.5 | 18 | 18 | 22.5 | 22.5 |
| Colloidal silica | | | | | | | 5.4 | 7.2 | 7.2 | 9.0 | 9.0 |
| Magnesium stearate | | | | | | | 2.7 | 3.6 | 3.6 | 4.5 | 4.5 |
| Weight | | | | | | | 270 | 360 | 360 | 450 | 450 |

### 8.2 Preparation

For formulas 31-32, the API and excipients with the formulation amount for 1000 tablets were mixed in a hopper mixer. The content was monitored on line. The mixture was filled in capsules. For formulas 33-34, after mixing, the mixture was directly pressed into raw tablets, which were then coated. The weight increase of coating was controlled by 3%. For formula 35, the compound of formula I and other excipients except magnesium stearate were mixed in a hopper mixer. The mixture was pressed into a ribbon by using a roller press machine, the ribbon was then crushed into granules. Magnesium stearate was added, and the mixture was blended for 10 min. The content was monitored. The mixture was pressed into tablets, which were then coated. The weight increase of coating was controlled by 3%.

### 8.3 Dissolution data

| | | | | | | | Formula 31 | Formula 32 | Formula 33 | Formula 34 | Formula 35 |
|---|---|---|---|---|---|---|---|---|---|---|---|
| 5 min | | | | | | | 85 | 77 | 76 | 83 | 70 |
| 15 min | | | | | | | 94 | 85 | 91 | 95 | 85 |
| 30 min | | | | | | | 98 | 100 | 97 | 99 | 99 |

### 8.4 Stability data

The capsules prepared in Example 8 were packaged in a commercially available package, and then placed under a relative humidity of 75% ± 5% at 40°C ± 2°C for 6 months. The results are shown in Table 1.

**Table 1: Results of stability test**

| Test items | | Formula 31 | | Formula 32 | | Formula 33 | | Formula 34 | | Formula 35 | |
|---|---|---|---|---|---|---|---|---|---|---|---|
| | | 0 day | 6M | 0 day | 6M | 0 day | 6M | 0 day | 6M | 0 day | 6M |
| | Content (%) | 99.52 | 99.78 | 99.46 | 99.41 | 100.23 | 99.43 | 99.51 | 99.42 | 99.51 | 99.42 |
| | Total impurity (%) | 0.18 | 0.17 | 0.19 | 0.18 | 0.18 | 0.19 | 0.17 | 0.18 | 0.17 | 0.18 |
| Dissolution rate (%) | 5 min | 85 | 87 | 80 | 80 | 80 | 79 | 87 | 82 | 70 | 72 |
| | 15 min | 95 | 95 | 90 | 88 | 91 | 89 | 95 | 95 | 85 | 86 |
| | 30 min | 99 | 98 | 100 | 99 | 97 | 98 | 99 | 98 | 99 | 98 |

### Example 9

### 9.1 Unit dosage formula

| | Formula 36 | Formula 37 | Formula 38 | Formula 39 | Formula 40 | Formula 41 |
|---|---|---|---|---|---|---|
| Salt of compound of formula I¹ | 81 | 81 | 81 | 160 | 160 | 160 |
| Microcrystalline cellulose | 101 | 67.5 | 135 | 100 | 56.5 | 33 |
| Lactose | 101.5 | 135 | 67.5 | 37.5 | 56.5 | 66 |
| Starch | / | / | / | / | / | / |
| Hydroxypropyl cellulose | / | / | / | / | 17.5 | 17.5 |
| Povidone | 17.5 | 17.5 | 17.5 | / | / | / |
| Sodium carboxymethyl starch | / | / | 35 | 17.5 | 17.5 | / |
| Crospovidone | 35 | 35 | / | / | 17.5 | 17.5 |
| Croscarmellose sodium | / | / | / | 17.5 | / | 35 |
| Sodium dodecyl sulfate | 3.5 | 3.5 | 3.5 | 7.0 | 14.0 | 10.5 |
| Colloidal silica | 3.5 | 3.5 | / | 7.0 | 7.0 | 7.0 |
| Talc | / | / | 3.5 | / | / | / |
| Glyceryl behenate | 7.0 | 7.0 | 7.0 | 3.5 | 3.5 | 3.5 |
| Weight | 350 | 350 | 350 | 350 | 350 | 350 |

| | | | | | | |
|---|---|---|---|---|---|---|
| Note 1: the conversion coefficient of the compound of formula I to hydrochloride is 447.53/484.03=92.4%, i.e., 75 mg of the compound of formula I corresponds to 81 mg of hydrochloride of the compound of formula I; the conversion coefficient of the compound of formula I to isethionate is 447.53/573.53=78.0%, i.e., 75 mg of compound of formula I corresponds to 96 mg of isethionate of the compound of formula I; other specifications are converted correspondingly. | | | | | | |

### 9.2 Preparation

Wet granulation was carried out with the salt of the compound of formula I and excipients except lubricant, the granule was sieved after drying, a lubricant was added, and the mixture was blended for 10 min. The content was monitored on line. For formulas 36-38, the mixture was filled in capsules; for formulas 39-41, the mixture was pressed into tablets, which were then coated, and the weight increase of coating was controlled by 3%.

### 9.3 Dissolution data

| | Formula 36 | Formula 37 | Formula 38 | Formula 39 | Formula 40 | Formula 41 |
|---|---|---|---|---|---|---|
| 5 min | 85 | 79 | 77 | 65 | 56 | 50 |
| 15 min | 95 | 89 | 90 | 79 | 80 | 76 |
| 30 min | 102 | 96 | 97 | 94 | 93 | 94 |

## Claims

1. A pharmaceutical composition of a compound of formula (I) or a salt thereof, comprising the compound of formula I or the salt thereof, and a pharmaceutically acceptable excipient as a carrier, wherein the salt is selected from the group consisting of hydrochloride and isethionate, 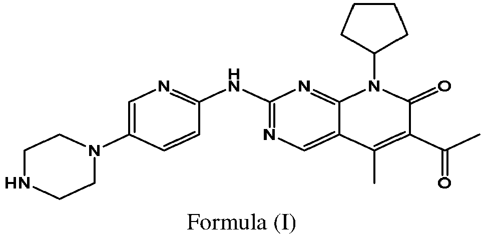 **characterized in that** the weight percentage of each component in the pharmaceutical composition is as follows:
| | |
|---|---|
| compound I or salt thereof | 20∼40% |
| disintegrant | 3∼15% |
| diluent | 55∼70% |
| lubricant | 0.1~3.5% |
| binder | 0∼8% |
| surfactant | 0∼5.0%, |
wherein the diluent is microcrystalline cellulose and lactose, and the weight ratio of lactose to microcrystalline cellulose is 1:2-2:1,
wherein the binder is at least one selected from the group consisting of hydroxypropyl cellulose and povidone, the disintegrant is at least one selected from the group consisting of crospovidone, sodium carboxymethyl starch, and croscarmellose sodium, the lubricant is at least one selected from the group consisting of magnesium stearate, colloidal silica, silica and talc, the surfactant is sodium dodecyl sulfate.

2. A preparation method of a pharmaceutical composition of a compound of formula (I) or a salt thereof according to claim 1, **characterized in that** the pharmaceutical composition is prepared by wet granulation, and the method comprises the following steps of:
1) pre-mixing the compound of formula I or the salt thereof with a part of excipient in a wet mixing granulator to obtain a pre-mixture;
2) adding a granulation liquid to granulate the pre-mixture obtained in step 1);
3) drying the granule obtained in step 2) in a fluidized bed dryer or a drying oven;
4) optionally, dry screening the dry granule obtained in step 3);
5) mixing the dry granule obtained in step 4) with the rest of excipient to obtain a final mixture;
6) optionally, filling the mixture obtained in the aforementioned step 5) by a suitable capsule filling machine to prepare a capsule;
7) optionally, pressing the mixture obtained in the aforementioned step 5) by a suitable tabletting machine to obtain a tablet core;
8) optionally, film coating the tablet core obtained in step 7) with a film coating.

3. The preparation method according to claim 2, **characterized in that** the granulation liquid is water.

4. A preparation method of a pharmaceutical composition of a compound of formula (I) or a salt thereof according to claim 1, **characterized in that** the pharmaceutical composition is prepared by dry granulation, and the method comprises the following steps of:
1) mixing the compound of formula I or the salt thereof with a part of excipient in a hopper mixer to obtain a pre-mixture;
2) pressing the mixture obtained in step 1) in a suitable roller press machine;
3) crushing the ribbon obtained during step 2) into a granule by a suitable grinding or screening step;
4) optionally, mixing the granule obtained in step 3) with the rest of excipient in a mixer to obtain a final mixture;
5) optionally, filling the mixture obtained in the aforementioned step 4) by a suitable capsule filling machine to prepare a capsule;
6) optionally, pressing the mixture obtained in the aforementioned step 4) by a suitable press tabletting to obtain a tablet core;
7) optionally, film coating the tablet core obtained in step 6) with a film coating.

5. A preparation method of a pharmaceutical composition of a compound of formula (I) or a salt thereof according to claim 1, **characterized in that** the pharmaceutical composition is prepared by a method of direct mixing.

6. The preparation method according to claim 5, **characterized in that** the method comprises the following steps of:
1) mixing the compound of formula I or the salt thereof with all other excipients in a hopper mixer to obtain a mixture;
2) optionally, filling the mixture obtained in the aforementioned step 1) by a capsule filling machine to prepare a capsule;
3) optionally, pressing the mixture obtained in the aforementioned step 1) by a suitable tabletting machine to obtain a tablet core;
4) optionally, film coating the tablet core obtained in step 3) with a film coating.

## Patentansprüche

1. Pharmazeutische Zusammensetzung einer Verbindung der Formel (I) oder eines Salzes davon, umfassend die Verbindung der Formel I oder das Salz davon und einen pharmazeutisch verträglichen Hilfsstoff als Träger, worin das Salz ausgewählt ist aus der Gruppe bestehend aus Hydrochlorid und Isethionat, 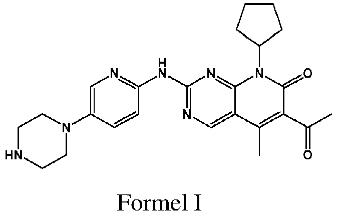 **dadurch gekennzeichnet, dass** die Gewichtsprozentsätze jeder Komponente in der pharmazeutischen Zusammensetzung wie folgt ist:
| | |
|---|---|
| Verbindung I oder Salz davon | 20-40% |
| Sprengmittel | 3-15% |
| Verdünnungsmittel | 55-70% |
| Schmiermittel | 0,1-3,5% |
| Bindemittel | 0-8% |
| Tensid | 0-5,0%, |
worin das Verdünnungsmittel mikrokristalline Cellulose und Lactose ist und das Gewichtsverhältnis von Lactose zu mikrokristalliner Cellulose 1:2-2:1 beträgt,
worin das Bindemittel mindestens eines ist ausgewählt aus der Gruppe bestehend aus Hydroxypropylcellulose und Povidon, worin das Sprengmittel mindestens eines ist ausgewählt aus der Gruppe bestehend aus Crospovidon, Natriumcarboxymethylstärke und Croscarmellose-Natrium, worin das Gleitmittel mindestens eines ist ausgewählt aus der Gruppe bestehend aus Magnesiumstearat, kolloidalem Siliziumdioxid, Siliziumdioxid und Talkum, und worin das Tensid Natriumdodecylsulfat ist.

2. Verfahren zur Herstellung einer pharmazeutischen Zusammensetzung einer Verbindung der Formel (I) oder eines Salzes davon nach Anspruch 1, **dadurch gekennzeichnet, dass** die pharmazeutische Zusammensetzung durch Nassgranulation hergestellt wird und dass das Verfahren die folgenden Schritte umfasst:
1) Vormischen der Verbindung der Formel I oder des Salzes davon mit einem Teil des Hilfsstoffs in einem Nassmischgranulator, um eine Vormischung zu erhalten;
2) Zugabe einer Granulierflüssigkeit zum Granulieren der in Schritt 1) erhaltenen Vormischung;
3) Trocknen des in Schritt 2) erhaltenen Granulats in einem Wirbelschichttrockner oder einem Trockenofen;
4) wahlweise Trockensieben des in Schritt 3) erhaltenen trockenen Granulats;
5) Mischen des in Schritt 4) erhaltenen trockenen Granulats mit dem Rest des Hilfsstoffs, um eine endgültige Mischung zu erhalten;
6) wahlweise Füllen der in dem vorgenannten Schritt 5) erhaltenen Mischung durch eine geeignete Kapselfüllmaschine, um eine Kapsel herzustellen;
7) wahlweise Pressen der in dem vorgenannten Schritt 5) erhaltenen Mischung durch eine geeignete Tablettiermaschine, um einen Tablettenkern zu erhalten;
8) wahlweise Beschichten des in Schritt 7) erhaltenen Tablettenkerns mit einem Filmüberzug.

3. Herstellungsverfahren nach Anspruch 2, **dadurch gekennzeichnet, dass** die Granulationsflüssigkeit Wasser ist.

4. Verfahren zur Herstellung einer pharmazeutischen Zusammensetzung einer Verbindung der Formel (I) oder eines Salzes davon nach Anspruch 1, **dadurch gekennzeichnet, dass** die pharmazeutische Zusammensetzung durch Trockengranulation hergestellt wird, und dass das Verfahren die folgenden Schritte umfasst::
1) Mischen der Verbindung der Formel I oder des Salzes davon mit einem Teil des Hilfsstoffs in einem Trichtermischer, um eine Vormischung zu erhalten;
2) Pressen der in Schritt 1) erhaltenen Mischung in einer geeigneten Walzenpressmaschine;
3) Zerkleinern des in Schritt 2) erhaltenen Bandes in ein Granulat durch einen geeigneten Mahl- oder Siebschritt;
4) wahlweise Mischen des in Schritt 3) erhaltenen Granulats mit dem Rest des Hilfsstoffs in einem Mischer, um eine endgültige Mischung zu erhalten;
5) wahlweise Füllen der in dem vorgenannten Schritt 4) erhaltenen Mischung durch eine geeignete Kapselfüllmaschine, um eine Kapsel herzustellen;
6) wahlweise Pressen der in dem vorgenannten Schritt 4) erhaltenen Mischung durch eine geeignete Pressentablettierung, um einen Tablettenkern zu erhalten;
7) wahlweise Beschichten des in Schritt 6) erhaltenen Tablettenkerns mit einem Filmüberzug.

5. Verfahren zur Herstellung einer pharmazeutischen Zusammensetzung einer Verbindung der Formel (I) oder eines Salzes davon nach Anspruch 1, **dadurch gekennzeichnet, dass** die pharmazeutische Zusammensetzung durch ein Direktmisch-Verfahren hergestellt wird.

6. Zubereitungsverfahren nach Anspruch 5, **dadurch gekennzeichnet, dass** das Verfahren die folgenden Schritte umfasst::
1) Mischen der Verbindung der Formel I oder des Salzes davon mit allen anderen Hilfsstoffen in einem Trichtermischer, um eine Mischung zu erhalten;
2) wahlweise Füllen der im vorgenannten Schritt 1) erhaltenen Mischung durch eine Kapselfüllmaschine zur Herstellung einer Kapsel;
3) wahlweise Pressen der in dem vorgenannten Schritt 1) erhaltenen Mischung durch eine geeignete Tablettiermaschine, um einen Tablettenkern zu erhalten;
4) wahlweise das Beschichten des in Schritt 3) erhaltenen Tablettenkerns mit einem Filmüberzug.

## Revendications

1. Composition pharmaceutique d'un composé de formule (I) ou d'un sel de celui-ci, comprenant le composé de formule I ou le sel de celui-ci, et un excipient pharmaceutiquement acceptable en tant que véhicule, dans laquelle le sel est choisi dans le groupe constitué par le chlorhydrate et l'iséthionate, 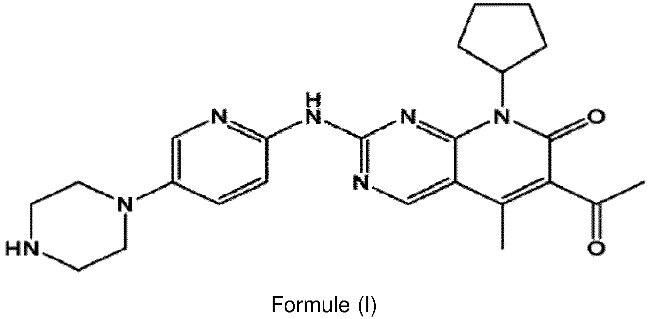 **caractérisée en ce que** le pourcentage en poids de chaque composant dans la composition pharmaceutique est comme suit :
| | |
|---|---|
| composé I ou sel de celui-ci | 20 à environ 40 % |
| délitant | 3 à environ 15 % |
| diluant | 55 à environ 70 % |
| lubrifiant | 0,1 à environ 3,5 % |
| liant | 0 à environ 8 % |
| tensioactif | 0 à environ 5,0 % |
dans laquelle le diluant est de la cellulose microcristalline et du lactose, et le rapport en poids du lactose à la cellulose microcristalline est de 1:2 à 2:1,
dans laquelle le liant est au moins un choisi dans le groupe constitué par l'hydroxypropylcellulose et la povidone, le délitant est au moins un choisi dans le groupe constitué par la crospovidone, le carboxyméthylamidon sodique et la croscarmellose sodique, le lubrifiant est au moins un choisi dans le groupe constitué par le stéarate de magnésium, la silice colloïdale, la silice et le talc, le tensioactif est le dodécylsulfate de sodium.

2. Procédé de préparation d'une composition pharmaceutique d'un composé de formule (I) ou d'un sel de celui-ci selon la revendication 1, **caractérisé en ce que** la composition pharmaceutique est préparée par granulation humide, et le procédé comprend les étapes suivantes consistant à :
1) pré-mélanger le composé de formule I ou le sel de celui-ci avec une partie des excipients dans un granulateur de mélange par voie humide pour obtenir un pré-mélange ;
2) ajouter un liquide de granulation pour granuler le pré-mélange obtenu à l'étape 1) ;
3) sécher le granulé obtenu à l'étape 2) dans un séchoir à lit fluidisé ou une étuve de séchage ;
4) éventuellement tamiser à sec le granulé sec obtenu à l'étape 3) ;
5) mélanger le granulé sec obtenu à l'étape 4) avec le reste des excipients pour obtenir un mélange final ;
6) éventuellement, charger le mélange obtenu à l'étape 5) mentionnée ci-dessus par le biais d'une machine de remplissage de gélules appropriée pour préparer une gélule ;
7) éventuellement, presser le mélange obtenu à l'étape 5) mentionnée ci-dessus par le biais d'une machine de fabrication de comprimés appropriée pour obtenir un noyau de comprimé ;
8) éventuellement, pelliculer le noyau du comprimé obtenu à l'étape 7) avec un pelliculage.

3. Procédé de préparation selon la revendication 2, **caractérisé en ce que** le liquide de granulation est l'eau.

4. Procédé de préparation d'une composition pharmaceutique d'un composé de formule (I) ou d'un sel de celui-ci selon la revendication 1, **caractérisé en ce que** la composition pharmaceutique est préparée par granulation à sec, et le procédé comprend les étapes suivantes consistant à :
1) mélanger le composé de formule I ou le sel de celui-ci avec une partie des excipients dans un mélangeur à trémie pour obtenir un pré-mélange ;
2) presser le mélange obtenu à l'étape 1) dans une presse à rouleaux appropriée ;
3) broyer le ruban obtenu pendant l'étape 2) en un granulé par une étape de broyage ou de tamisage appropriée ;
4) éventuellement, mélanger le granulé obtenu à l'étape 3) avec le reste des excipients dans un mélangeur pour obtenir un mélange final ;
5) éventuellement, charger le mélange obtenu à l'étape 4) mentionnée ci-dessus par le biais d'une machine de remplissage de gélules appropriée pour préparer une gélule ;
6) éventuellement, presser le mélange obtenu à l'étape 4) mentionnée ci-dessus par le biais d'une machine de fabrication de comprimés appropriée pour obtenir un noyau de comprimé ;
7) éventuellement, pelliculer le noyau du comprimé obtenu à l'étape 6) avec un pelliculage.

5. Procédé de préparation d'une composition pharmaceutique d'un composé de formule (I) ou d'un sel de celui-ci selon la revendication 1, **caractérisé en ce que** la composition pharmaceutique est préparée par un procédé de mélange direct.

6. Procédé de préparation selon la revendication 5, **caractérisé en ce que** le procédé comprend les étapes suivantes consistant à :
1) mélanger le composé de formule I ou le sel de celui-ci avec tous les autres excipients dans un mélangeur à trémie pour obtenir un mélange ;
2) éventuellement, charger le mélange obtenu à l'étape 1) mentionnée ci-dessus par le biais d'une machine de remplissage de gélules appropriée pour préparer une gélule ;
3) éventuellement, presser le mélange obtenu à l'étape 1) mentionnée ci-dessus par le biais d'une machine de fabrication de comprimés appropriée pour obtenir un noyau de comprimé ;
4) éventuellement, pelliculer le noyau du comprimé obtenu à l'étape 3) avec un pelliculage.
